# EUROPÄISCHE PATENTSCHRIFT

(11) **EP 0 839 500 B1**
(45) Veröffentlichungstag und Bekanntmachung des Hinweises auf die Patenterteilung: **28.02.2001**
(21) Anmeldenummer: 96120473.2
(22) Anmeldetag: 19.12.1996
(51) Int. Cl.: A61B 17/16, A61F 2/28

(54) **Knochenraspel**
Bone rasp
Râpe à os

(30) Priorität: 31.10.1996 DE 19644015
(43) Veröffentlichungstag der Anmeldung: 06.05.1998
(73) Patentinhaber: Quétin, Roswitha, 69181 Leimen (DE)
(72) Erfinder: Quétin, Roswitha, 69181 Leimen (DE)
(74) Vertreter: Hilleringmann, Jochen, Dipl.-Ing.

(56) Entgegenhaltungen:
- DE-C- 3 734 714
- FR-A- 2 712 483
- US-A- 5 147 407

## Beschreibung

Die Erfindung betrifft eine Knochenraspel, wie sie beispielsweise von Implantologen, Paradontologen, (Oral-) Chirurgen und Zahnärzten benötigt wird.

Bei der Implantation von Zähnen ist es mitunter erforderlich, seitlich des Kieferknochens Knochenmaterial anzusetzen, damit der implantierte Zahn allseitig von Knochen umgeben ist. Das zu ergänzende Knochenmaterial wird dem Patienten entnommen und mittels einer Zange manuell zerkleinert. Bei dieser Zerkleinerungsmethode besteht das Problem darin, daß die vom entnommenen Knochenmaterial absplitternden Teile aufgefangen werden müssen. Angesichts der Tatsache, daß die Menge an dem Patienten zu entnehmenden Knochenmaterial so gering wie möglich sein sollte, entsteht also bei der manuellen Zerkleinerungsmethode mittels Zange die Gefahr, daß relativ große Mengen an Knochenmaterial entnommen werden müssen, um nach dem Zerkleinerungsvorgang noch eine ausreichende Menge an zerkleinertem Material zur Verfügung zu haben, da ein relativ großer Anteil verloren gegangen ist (umherfliegende Partikel, die nicht aufgefangen werden konnten).

Eine andere im Stand der Technik bekannte Methode der Zerkleinerung entnommenen Knochenmaterials besteht darin, sich eines speziellen Raspelwerks zu bedienen. Das bekannte System weist einen mit radial verlaufenden geradlinigen Schneidkanten versehenen Schneidteller auf, der drehfest mit einer Spindel verbunden ist. Die Spindel steht in Gewindeeingriff mit einem zylindrischen Gehäuse und läßt sich von außen manuell mittels eines Betätigungswerkzeuges verdrehen. Das Betätigungswerkzeug weist einen Innensechskant auf, der auf einen an dem dem Teller abgewandten Ende vorgesehenen Außensechskant der Spindel aufsetzbar ist. Durch Verdrehen der Spindel bewegt sich der mit Schneidkanten versehene Teller axial in Richtung auf einen Siebboden des zylindrischen Gehäuses zu. Unterhalb des Siebbodens ist eine Auffangkappe angeordnet. Durch Verdrehen der Spindel drückt der Schneidteller allmählich immer stärker gegen zwischen dem Schneidteller und dem Siebboden angeordnetes Knochenmaterial. In der Anfangsphase der Kontaktierung des Schneidtellers und des Knochenmaterials wird möglicherweise noch Knochenmaterial zerkleinert. Im laufe der weiteren Bewegung des Schneidtellers kommt es jedoch zunehmend zu einer Druck- und Preßeinwirkung auf das Knochenmaterial, was die weitere Verdrehung des Schneidtellers nahezu unmöglich macht und insbesondere nicht mehr zu einer Zerkleinerung durch Abtragung von einzelnen Spänen vom Knochenmaterial führt.

Eine gattungsgemäße Knochenraspel ist aus FR-A-2712483 bekannt, wobei das Raspelwerkzeug trommelförmig ausgebildet ist und eine Zylinderwand mit Löchern aufweist. Die jeweiligen Kanten der Löcher sind scharf und gewährleisten die Zerkleinerung des in einer radialen Aufnahme eingepreßten Knochenmaterials.

Der Erfindung liegt die Aufgabe zugrunde, eine Knochenraspel zum Zerkleinern von Knochenmaterial zu schaffen, die einfach handhabbar ist und mittels derer sich beim Zerkleinerungsvorgang lediglich geringste nicht ins Gewicht fallende Verlustmengen an geraspeltem Knochenmaterial einstellen.

Zur Lösung dieser Aufgabe wird mit der Erfindung eine Knochenraspel vorgeschlagen, die versehen ist mit
- einem drehbaren trommelförmigen Raspelwerkzeug mit einer Zylinderwand und
- einem Gehäuse, in dem ein zylindrischer Aufnahmeraum für das Raspelwerkzeug und ein sich vom Aufnahmeraum radial erstreckender Zuführschacht für Knochenstücke ausgebildet ist, wobei
- das Raspelwerkzeug in seiner Zylinderwand eine Vielzahl von in mehreren axial verlaufenden Reihen nebeneinander angeordnete Durchgangsbohrungen aufweist,
- die Zylinderwand mehrere Zähne aufweist, die jeweils zwischen zwei benachbarten Durchgangsbohrungen angeordnet sind und in radialer Erstreckung des Raspelwerkzeuges betrachtet über die ihnen vorgelagerten Bereiche der Zylinderwand überstehen,
- die Zähne in Draufsicht auf die Zylinderwand betrachtet im wesentlichen V-förmig spitz zulaufend mit in Drehrichtung des Raspelwerkzeuges weisender Spitze ausgebildet sind und die Spitzen benachbarter Zähne unter Bildung einer abschnittsweise bogenförmigen und spitzen Schneidkante verbunden sind,
- die bogenförmigen Abschnitte in Drehrichtung des Raspelwerkzeuges betrachtet unmittelbar hinten den Durchgangsbohrungen angeordnet sind und
- die Schneidkanten von in Drehrichtung des Raspelwerkzeuges aufeinanderfolgenden Durchgangsbohrungsreihen unterschiedliche radiale Überstände über die Zylinderwand aufweisen.

Die erfindungsgemäße Knochenraspel gleicht in gewisser Weise einer Küchenreibe, bei der sich das Reib- bzw. Raspelwerkzeug relativ zum zu raspelnden Gut bewegt. Die Knochenmühle weist ein trommelförmiges Raspelwerkzeug mit einer Zylinderwand auf, das im Aufnahmeraum eines Gehäuses drehbar untergebracht ist. Mit dem Aufnahmeraum steht ein Zuführschacht in Verbindung, der sich radial zum zylindrischen Aufnahmeraum erstreckt. In diesen Zuführschacht wird zu raspelndes Knochenmaterial eingeführt, welches dann auf der Außenfläche der Zylinderwand des Raspelwerkzeuges aufliegt. Mittels eines Stempels kann das Knochenmaterial mit Kraft gegen die Zylinderwand radial gedrückt werden.

Das Raspelwerkzeug weist eine Vielzahl von Durchgangsbohrungen auf, die sich durch seine Zylinderwand hindurch erstrecken. Diese Durchgangsbohrungen sind jeweils in axial verlaufenden Reihen nebeneinanderliegend angeordnet. Zwischen den Durchgangsbohrungen befinden sich außen auf der Zylinderwand angeordnete Zähne, die in Draufsicht auf die Zylinderwand betrachtet, V-förmig spitz zulaufend ausgebildet sind, wobei die Spitze der Zähne in Drehrichtung des Raspelwerkzeuges weist. Die in Drehrichtung weisenden Spitzen der Zähne liegen dabei pro Reihe vorzugsweise auf gleicher Höhe (auf gleicher parallel zur axialen Erstreckung verlaufenden Linie). Vorzugsweise befinden sich die Spitzen der Zähne auf einer gemeinsamen Linie mit den Mittelpunkten der zwischen den Zähnen angeordneten Durchgangsbohrungen. Es ist aber auch möglich, die gemeinsame Linie, auf der die Spitzen der Zähne angeordnet sind, in Drehrichtung des Raspelwerkzeuges betrachtet relativ zu den Mittelpunkten der Durchgangsbohrungen nach hinten zu verlagern, so daß sie insbesondere etwa in Höhe des halben Radius der Durchgangsbohrungen angeordnet sind.

Die nebeneinanderliegenden und durch Durchgangsbohrungen voneinander getrennten Spitzen der Zähne sind durch bogenförmige Abschnitte untereinander verbunden. Hierdurch entstehen auf der Außenseite der Zylinderwand des Raspelwerkzeuges eine Vielzahl von Schneidkanten, die spitz zulaufende Abschnitte der Zähne und diese verbindende bogenförmige Abschnitte aufweisen. Insbesondere handelt es sich bei den bogenförmigen Abschnitten um Teilkreisabschnitte.

Die auf die obige Weise ausgebildeten Schneidkanten auf der Außenseite der Zylinderwand weisen untereinander unterschiedliche Höhe auf. D.h., daß in Drehrichtung des Raspelwerkzeuges aufeinanderfolgende Schneidkanten einen unterschiedlichen Überstand über die unmittelbar vor ihnen liegenden Bereiche der Zylinderwand aufweisen. Zweckmäßigerweise variiert der Höhenversatz zwischen zwei in Drehrichtung des Raspelwerkzeuges aufeinanderfolgende Schneidkanten alternierend.

Die Durchgangsbohrungen sind bei der erfindungsgemäßen Knochenraspel in unmittelbarer Nähe der Schneidkanten, wobei die bogenförmigen Abschnitte der Schneidkanten tangential zu den Rändern der Durchgangsbohrungen verlaufen, d.h. diese über einen Teilwinkelbereich bilden. Die in Drehrichtung des Raspelwerkzeuges weisenden steilen Flanken der Schneidkanten verlaufen zumindest radial, vorzugsweise in einem spitzen Winkel zur Radialerstreckung, so daß sich durch die Schneidkanten ein Hinterschnitt ergibt.

Mit einer Knochenraspel der vorstehend genannten erfindungsgemäßen Art ließen sich bei Testversuchen erstaunliche Ergebnisse erzielen. So war es beispielsweise möglich, das härteste menschliche Knochenmaterial aus dem vorderen Bereich des Unterkiefers (corticalis compacta) unter Einsatz lediglich geringer Kräfte zu zerspanen bzw. zu zerraspeln. Die Menge an am Raspelwerkzeug verbleibendem Knochenmaterial war relativ zum Raspelgut betrachtet verschwindend gering; insoweit weist die erfindungsgemäße Knochenraspel also einen hohen Nutzungsgrad von nahezu 1 (Verhältnis aus zu raspelndem Knochenmaterial zu geraspeltem Knochenmaterial) auf.

In vorteilhafter Weiterbildung der Erfindung sind die Durchgangsbohrungen geringfügig versetzt zur Radialerstreckung in die Zylinderwand des Raspelwerkzeuges eingebracht. Bei einem Außendurchmesser des Raspelwerkzeuges von 3 bis 5 cm beträgt der Versatz vorzugsweise 1,5 mm.

Der Höhenversatz benachbarter Schneidkanten beträgt bei einem Raspelwerkzeug mit einem Außendurchmesser von etwa 3 bis 5 cm vorzugsweise 1/10 mm. Die niedrigeren Schneidkanten betragen dabei insbesondere 0,25 mm, so daß die höheren Schneidkanten demzufolge 0,35 mm Überstand aufweisen. Es hat sich herausgestellt, daß es ausreichend ist, lediglich zwei unterschiedlich hohe Schneidkanten auf der Außenseite der Zylinderwand des Raspelwerkzeuges vorzusehen, wobei diese Schneidkanten alternierend in Drehrichtung des Raspelwerkzeuges betrachtet aufeinanderfolgen.

Um das Raspelgut zuverlässig und bequem auffangen zu können, ist gemäß einer Weiterentwicklung der Erfindung vorgesehen, am Gehäuse eine Auffangkappe anzuordnen, die lösbar mit dem Gehäuse verbindbar ist und im angebrachten Zustand den das Raspelwerkzeug aufnehmenden Aufnahmeraum an seinem einen axialen Ende zu verschließen.

Das Raspelwerkzeug weist vorzugsweise eine axial abstehende Antriebswelle auf, die mit einem Handknebel verbindbar ist. Ein derartiger Handknebel hat sich bei Versuchen als einfach zu bedienendes Betätigungsorgan zum manuellen Drehen des Raspelwerkzeuges herausgestellt. Insbesondere im Vergleich zu einer Handkurbel weist ein Handknebel, der zwei diametral gegenüberliegende und radial überstehende Arme aufweist, Vorteile auf, wenn die Maßtoleranzen der Knochenraspel extrem gering sind. Mit einem derartigen Handknebel wird nämlich ein "Festfressen" der Lagerwelle des Raspelwerkzeuges eher verhindert, so daß zur Lagerung der Welle am Gehäuse keinerlei aufwendige Rollen- bzw. Wälzkörperlager eingesetzt werden müssen.

Vorzugsweise ist das Gehäuse der erfindungsgemäßen Knochenraspel mit einem klammerartigen Sockel mit verstellbarer Klammer versehen, um die Knochenraspel an einer Tischplatte o.dgl. Kante anbringen zu können. Im festgeklemmten Zustand der Knochenraspel verläuft das Raspelwerkzeug vorzugsweise zur Auffangkappe hin geneigt.

Alternativ zu einem handbetätigbaren Drehorgan zur Drehung des Raspelwerkzeuges kann auch ein Motor eingesetzt werden.

Die erfindungsgemäße Knochenraspel ist im Rahmen dieser Beschreibung anhand eines trommelförmigen Raspelwerkzeuges beschrieben. Die erfindungsgemäße Ausbildung und Anordnung der Zähne des Raspelwerkzeuges läßt sich aber ebenso gut auch auf einer Teller- bzw. Kreisfläche realisieren. Über einen exzentrisch zur Achse des Tellers angeordneten Schacht wird dann Knochenmaterial zugeführt, das bei Rotation des Tellers zermahlen wird und in einen unterhalb des Tellers angeordneten Aufnahmeraum fällt. Die Durchgangsbohrungen durchdringen den Teller in axialer Richtung und sind insbesondere entlang radialer Linien angeordnet. Die Ausbildung der Schneidkanten ist gleich derjenigen gemäß obiger Beschreibung.

Nachfolgend wird anhand der Figuren ein Ausführungsbeispiel der Erfindung näher erläutert. Im einzelnen zeigen:
- Fig. 1: eine Seitenansicht einer Knochenraspel mit auf den überstehenden Rand einer Platte aufgesetztem Klemmfuß,
- Fig. 2: eine Draufsicht in radialer Richtung auf das Raspelwerkzeug,
- Fig. 3: einen Schnitt entlang der Linie III-III der Fig. 2,
- Fig. 4: eine vergrößerte Darstellung des in Fig. 3 mit IV gekennzeichneten Bereichs und
- Fig. 5: eine Draufsicht auf das Raspelwerkzeug gemäß V der Fig. 4.

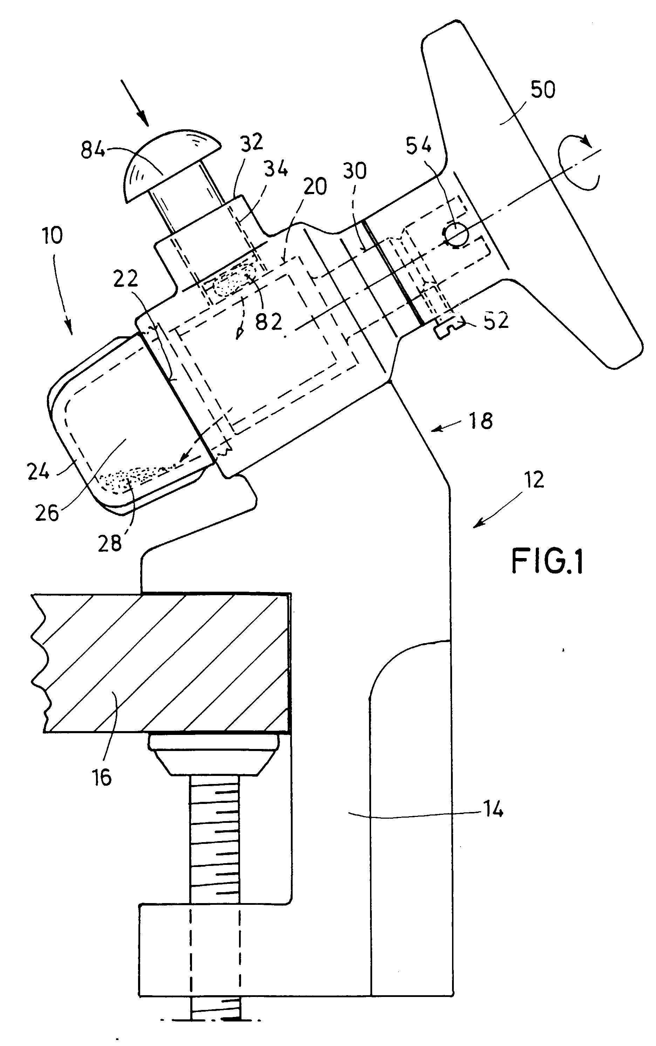

Fig. 1 zeigt in Seitenansicht eine Raspel 10 für Knochenmaterial. Die Raspel 10 weist ein Gehäuse 12 auf, das aus Metall, insbesondere Edelstahl, besteht und einen Klemmfuß 14 aufweist. Mittels des Klemmfußes 14 läßt sich das Gehäuse 12, wie in Fig. 1 gezeigt, an dem freiliegenden Rand einer beispielsweise Tisch- oder Arbeitsplatte 16 festklemmen.

Oberhalb des Klemmfußes 14 ist das Gehäuse 12 abgewinkelt. In diesem abgewinkelten Bereich 18 ist das Gehäuse 12 mit einem zylindrischen Aufnahmeraum 20 versehen, der zu einem axialen Ende 22 hin offen ist. In diesem Bereich läßt sich auf das Gehäuse 12 eine Schraubkappe 24 aufschrauben, die eine Innenmulde 26 zur Aufnahme von Raspelgut 28 aufweist.

Auf der der Schraubkappe 24 abgewandten Seite ist der Aufnahmeraum 20 mit einer koaxialen Durchgangsöffnung 30 verbunden, die auf der der Schraubkappe 24 gegenüberliegenden Seite des abgewinkelten Bereichs 18 des Gehäuses 12 endet. Radial zum zylindrischen Aufnahmeraum 20 erstreckt sich auf dem dem Klemmfuß 14 abgewandten oberen Ende 32 des Gehäuses 12 ein Einfüll- bzw. Zuführschacht 34, der sich bis zum oberen Ende 32 des Gehäuses 12 erstreckt.

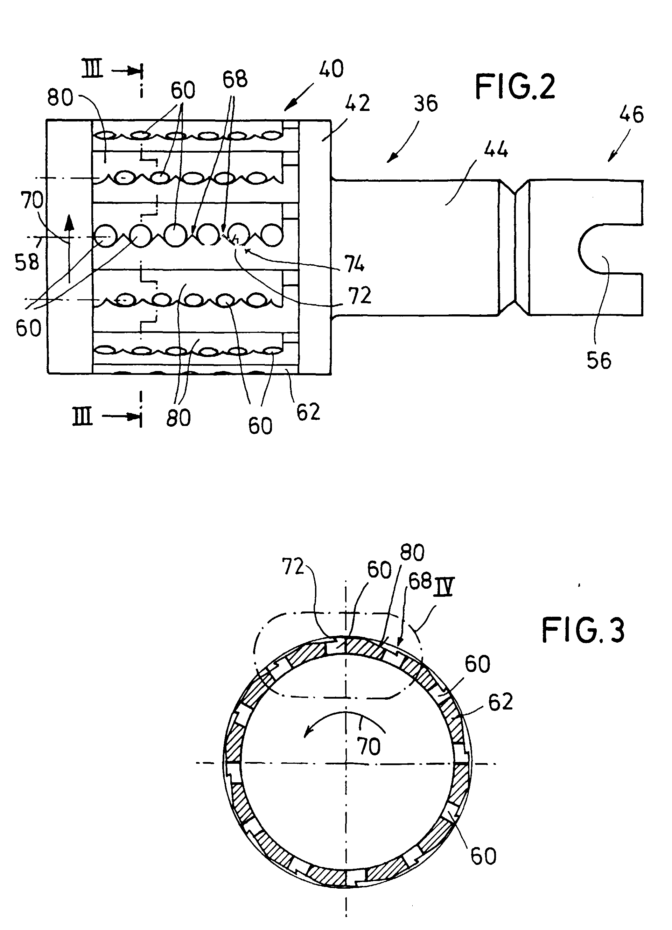

Über das offene axiale Ende 22 des Aufnahmeraums 20 läßt sich in diesen ein Raspelwerkzeug 36 einführen; eine Darstellung dieses Raspelwerkzeuges ist in Fig. 2 wiedergegeben. Das Raspelwerkzeug 36 weist einen einseitig offenen Trommelteil 40 mit einer an das geschlossene Ende 42 sich anschließenden koaxialen Welle 44 auf. Diese Welle 44 erstreckt sich im eingebauten Zustand des Raspelwerkzeuges 36 durch die Durchgangsöffnung 30 des Gehäuses 12 hindurch und ragt über dieses über. Auf das überstehende Ende 46 wird dann ein Handknebel 50 geschoben, der gegen axiale Verschiebungen über eine radiale Klemmschraube 52 an der Welle 44 festgelegt werden kann. Die verdrehsichere Kopplung von Handknebel 50 und Welle 44 erfolgt durch einen radialen Bolzen 54 des Handknebels 50, der in eine radiale durchgehende Nut 56 der Welle 44 eintaucht.

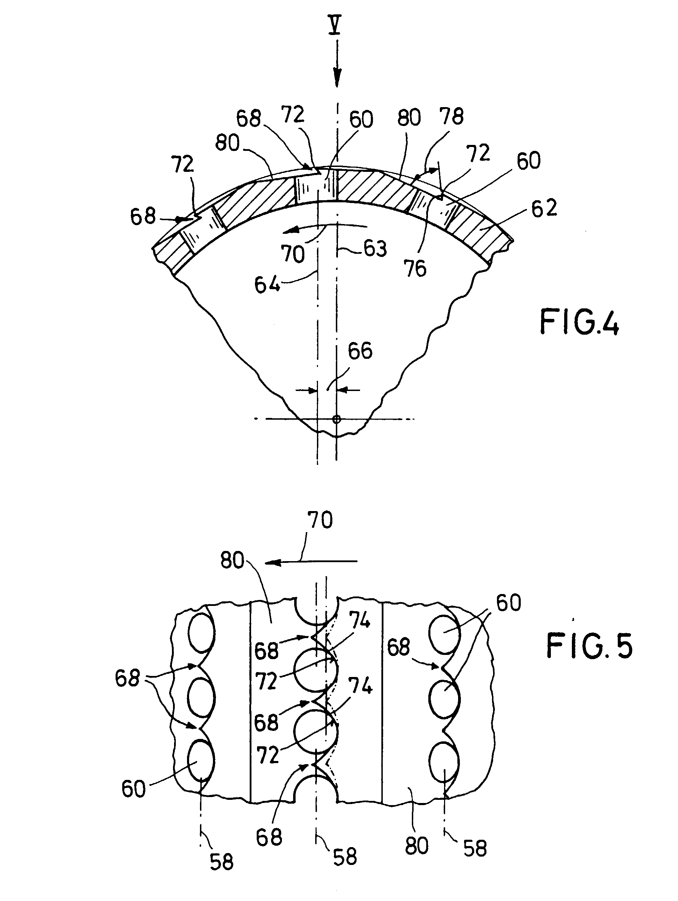

Gemäß den Fign. 2 bis 5 weist der Trommelteil 40 des Raspelwerkzeuges 36 mehrere entlang axial verlaufender Reihen 58 angeordnete Durchgangsbohrungen 60 auf, die in die zylindrische Wand 62 des Trommelteils 40 eingebracht sind. Wie insbesondere in Fig. 4 zu erkennen ist, sind die Bohrungen 60 in Umfangsrichtung versetzt zur Radialerstreckung (siehe Linie 63 der Fig. 4) angeordnet, so daß zwischen den Mittelachsen 64 der Durchgangsbohrungen 60 und der Radialerstreckung 63 ein Versatz 66 entsteht.

Außen auf der Zylinderwand 62 befinden sich eine Vielzahl von spitz zulaufenden im wesentlichen V-förmigen Erhebungen 68, die in Drehrichtung 70 des Raspelwerkzeuges 36 weisen. Diese spitzen Zähne 68 sind Bestandteile von wellenförmigen Schneidkanten 72, die zwischen den spitzen Zähnen 68 kreisbogenförmige Abschnitte 74 aufweisen. Im Bereich dieser kreisbogenförmigen Abschnitte 74 bilden die Schneidkanten 72 über einen gewissen Umfangsteil den Rand der Bohrungen 60. Anhand von Fig. 4 ist insbesondere zu erkennen, daß die Höhe der Schneidkanten 72 von Schneidkante zu Schneidkante alternierend variiert. Ferner ergibt sich aus der Darstellung gemäß Fig. 4, daß die Schneidkanten 72 Hinterschnitte aufweisen, d.h. daß ihre steilen Flanken 76 einen spitzen Winkel 78 mit den in Rotationsrichtung 70 betrachtet unmittelbar vor den Schneidkanten 72 und den Bohrungen 60 angeordneten Bereichen 80 der Zylinderwand 62 bilden. Die Bereiche 80 sind durch Abflachung der zylindrischen Außenseite der Zylinderwand 62 hergestellt.

Wie in Fig. 5 angedeutet, kann die Lage der spitzen Zähne 68 relativ zur Ausrichtung der Reihen 58 der Bohrungen 60 variieren. Gemäß Fig. 5 ist mit durchgezogenen Linien der Fall dargestellt, daß die spitzen Enden der Zähne 68 auf einer gemeinsamen Linie mit den Mittelpunkten der Bohrungen 60 angeordnet sind. Es ist aber auch der Fall denkbar, daß die Zähne 68 in Rotationsrichtung 70 betrachtet den Mittelpunkten der Bohrungen 60 nacheilen.

Zum Raspeln von Knochenmaterial werden Knochenstücke 82 in den Zuführschacht 34 eingebracht und mittels eines Stempels 84 gegen die sich drehende mit den erhabenen Zähnen 68 und den Schneidkanten 72 versehene Außenseite der Zylinderwand 62 des Trommelteils 40 des Raspelwerkzeuges 36 gedrückt. Das Raspelgut 28 wird über die den Schneidkanten 72 unmittelbar vorgelagerten Durchgangsbohrungen 60 ins Innere des Trommelteils 40 des Raspelwerkzeuges 36 transportiert. Aufgrund der Neigung des Raspelwerkzeuges 36 zum offenen axialen Ende 22 des Aufnahmeraums 20 des Gehäuses 12 gelangt das Raspelgut 28 in die Schraub- und Auffangkappe 24.

## Patentansprüche

1. Knochenraspel mit
- einem drehbaren trommelförmigen Raspelwerkzeug (36) mit einer Zylinderwand (62) und
- einem Gehäuse (12), in dem ein zylindrischer Aufnahmeraum (20) für das Raspelwerkzeug (36) und ein sich vom Aufnahmeraum (20) radial erstreckender Zuführschacht (34) für Knochenstücke ausgebildet ist, wobei
- das Raspelwerkzeug (36) in seiner Zylinderwand (62) eine Vielzahl von in mehreren axial verlaufenden Reihen (58) nebeneinander angeordnete Durchgangsbohrungen (60) aufweist,
- die Zylinderwand (62) mehrere Zähne (68) aufweist, die jeweils zwischen zwei benachbarten Durchgangsbohrungen (60) angeordnet sind und in radialer Erstreckung des Raspelwerkzeuges (36) betrachtet über die ihnen vorgelagerten Bereiche (80) der Zylinderwand (62) überstehen,
- die Zähne (68) in Draufsicht auf die Zylinderwand (62) betrachtet im wesentlichen V-förmig spitz zulaufend mit in Drehrichtung (70) des Raspelwerkzeuges (36) weisender Spitze ausgebildet sind und die Spitzen benachbarter Zähne (68) unter Bildung einer abschnittsweise bogenförmigen und spitzen Schneidkante (72) verbunden sind,
- die bogenförmigen Abschnitte (74) in Drehrichtung (70) des Raspelwerkzeuges (36) betrachtet unmittelbar hinten den Durchgangsbohrungen (60) angeordnet sind und
- die Schneidkanten (72) von in Drehrichtung (70) des Raspelwerkzeuges (36) aufeinanderfolgenden Durchgangsbohrungsreihen (58) unterschiedliche radiale Überstände über die Zylinderwand (62) aufweisen.

2. Knochenraspel nach Anspruch 1, dadurch gekennzeichnet, daß der Überstand der Schneidkanten (72) wechselweise unterschiedlich ist.

3. Knochenraspel nach Anspruch 1 oder 2, dadurch gekennzeichnet, daß die Durchgangsbohrungen (60) versetzt zur Radialerstreckung in der Zylinderwand (62) des Raspelwerkzeuges (36) eingebracht sind.

4. Knochenraspel nach einem der Ansprüche 1 bis 3, dadurch gekennzeichnet, daß die in Drehrichtung (70) des Raspelwerkzeuges (36) weisenden vorderen Kanten der Zähne (68) einen Hinterschnitt bilden.

5. Knochenraspel nach einem der Ansprüche 1 bis 4, dadurch gekennzeichnet, daß die Zähne (68) bzw. die Durchgangsbohrungen (60) von Reihe (58) zu Reihe (58) zueinander axial versetzt sind.

6. Knochenraspel nach einem der Ansprüche 1 bis 5, dadurch gekennzeichnet, daß mit dem Gehäuse (12) eine Auffangkappe (24) zum Auffangen des Raspelguts (28) lösbar verbindbar ist.

## Claims

1. A bone rasp comprising
- a rotatable drum-shaped rasp tool (36) with a cylinder wall (62), and
- a housing (12) provided with a cylindrical reception chamber (20) for the rasp tool (36) and with a supply well (34) for bone pieces extending radially from the reception chamber (20), wherein
- the rasp tool (36) has its cylinder wall (62) provided with a plurality of through bores (60) arranged adjacent to each other in a plurality of axially extending rows (58),
- the cylinder wall (62) comprises several teeth (68) respectively arranged between two adjacent through bores (60) and, when viewed in the radial extension of the rasp tool (36), projecting beyond the portions (80) of the cylinder wall (62) located in front of said teeth,
- the teeth (68), when seen in plan view of the cylinder wall (62), are substantially tapered in V-shape, the tip pointing in the rotational direction (70) of the rasp tool (36), and the tips of adjacent teeth (68) are connected while forming a cutting edge (72) with arcuate and pointed sections,
- the arcuate sections (74) are arranged directly behind the through bores (60) when viewed in the rotational direction (70) of the rasp tool (36), and
- the cutting edges (72) of through bore rows (58) succeeding each other in the rotational direction (70) of the rasp tool (36) radially project in different lengths beyond the cylinder wall (62).

2. The bone rasp according to claim 1, characterized in that the projecting length of the cutting edges (72) is alternately different.

3. The bone rasp according to claim 1 or 2, characterized in that the through bores (60) are formed in the cylinder wall (62) of the rasp tool (36) so as to be offset with respect to the radial extension.

4. The bone rasp according to any one of claims 1 to 3, characterized in that the front edges of the teeth (68) pointing in the rotational direction (70) of the rasp tool (36) form an undercut.

5. The bone rasp according to any one of claims 1 to 4, characterized in that the teeth (68) and the through bores (60), respectively, are axially offset with respect to each other from one row (58) to the other (58).

6. The bone rasp according to any one of claims 1 to 5, characterized in that a collection cap (24) for collecting the rasp material (28) is releasably connectable with the housing (12).

## Revendications

1. Râpe à os comprenant
- un outil de râpage (36) en forme de tambour rotatif comportant une paroi cylindrique (62), et
- un boîtier (12), dans lequel sont formés une chambre cylindrique (20) de logement pour l'outil de râpage (36) et un conduit d'amenée (34) pour des morceaux d'os, qui s'étend radialement à partir de la chambre de logement (20),
et dans lequel
- l'outil de râpage (36) comporte, dans sa paroi cylindrique (32), une multiplicité de perçages traversants (60) qui sont disposés côte-à-côte suivant plusieurs rangées axiales (58),
- la paroi cylindrique (62) comporte plusieurs dents (68), qui sont disposées respectivement entre deux perçages traversants voisins (60) et, lorsque l'on considère l'étendue radiale de l'outil de râpage (36), font saillie au-delà des parties (80) de la paroi cylindrique (62), qui sont situées devant elle,
- lorsqu'on regarde selon une vue en plan la paroi cylindrique (62), les dents (68) sont réalisées avec une forme pointue essentiellement en forme de V, dont la pointe est dirigée dans le sens de rotation (70) de l'outil de râpage (36), et les pointes de dents voisines (68) sont réunies moyennant la formation d'une arête de coupe (72) pointue et de forme arquée suivant des sections,
- lorsqu'on regarde dans le sens de rotation (70) de l'outil de râpage (36), les sections de forme arquée (74) sont disposées directement en arrière des perçages traversants (60), et
- les arêtes de coupe (72) de rangées (58) de perçages traversants, qui se succèdent dans le sens de rotation (70) de l'outil de râpage (36), présentent des parties saillantes radiales débordant de la paroi (62) du cylindre.

2. Râpe à os selon la revendication 1, caractérisée en ce que le débordement des arêtes de coupe (72) est alternativement différent.

3. Râpe à os selon la revendication 1 ou 2, caractérisée en ce que les perçages traversants (60) sont aménagés, d'une manière décalée par rapport à l'étendue radiale, dans la paroi cylindrique (62) de l'outil de râpage (36).

4. Râpe à os selon l'une des revendications 1 à 3, caractérisée en ce que les bords avant des dents (68), qui sont tournés dans le sens de rotation (70) de l'outil de râpage (36), forment une partie en contre-dépouille.

5. Râpe à os selon l'une des revendications 1 à 4, caractérisée en ce que les dents (68) et les perçages traversants (60) sont décalés axialement entre eux d'une rangée (58) à une autre rangée (58).

6. Râpe à os selon l'une des revendications 1 à 5, caractérisée en ce qu'un capuchon de réception (24) servant à recevoir le matériau râpé (28) peut être relié de façon amovible au boîtier (12).
